# EUROPEAN PATENT SPECIFICATION

(11) **EP 2 488 157 B1**
(45) Date of publication and mention of the grant of the patent: **30.12.2020**
(21) Application number: 10768760.0
(22) Date of filing: 15.10.2010
(51) Int. Cl.: A61K 9/00, A61K 31/167, A61K 31/56, A61P 11/02, A61K 31/573, A61K 31/045, A61K 45/06, A61P 43/00

(54) **IMPROVED FORMULATIONS**
VERBESSERTE FORMULIERUNGEN
FORMULATIONS AMÉLIORÉES

(30) Priority: 16.10.2009 GB 0918150
(43) Date of publication of application: 22.08.2012
(73) Proprietor: Jagotec AG, 4132 Muttenz (CH)
(72) Inventor: MUELLER-WALZ, Rudi, 79650 Schopfheim (DE); FUEG, Lise-Marie, 4144 Arlesheim (CH)
(74) Representative: Mathys & Squire
(86) International application number: PCT/EP2010/065569
(87) International publication number: WO 2011/045429

(56) References cited:
- WO-A1-02/078671
- WO-A1-2004/019985
- WO-A1-2005/034911
- WO-A2-02/30394
- US-A1- 2002 025 299
- US-A1- 2006 257 324
- GUPTA R K ET AL: "AN EVALUATION OF SALMETEROL IN THE TREATMENT OF CHRONIC OBSTRUCTIVE PULMONARY DISEASES", INDIAN JOURNAL OF CHEST DISEASES AND ALLIED SCIENCES, VALLABHBHAI PATEL CHEST INSTITUTE, NEW DELHI, IN, vol. 44, no. 3, 1 January 2002 (2002-01-01), pages 165-172, XP008018997, ISSN: 0377-9343
- MIRAVITLLES M ET AL: "TREATMENT AND QUALITY OF LIFE IN PATIENTS WITH CHRONIC OBSTRUCTIVE PULMONARY DISEASE", QUALITY OF LIFE RESEARCH, RAPID COMMUNICATIONS OF OXFORD, OXFORD, GB, vol. 11, no. 4, 1 January 2002 (2002-01-01), pages 329-338, XP008018999, ISSN: 0962-9343, DOI: DOI:10.1023/A:1015520110663
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: DOI:10.1081/DIS-120015368

## Description

The present invention relates to pharmaceutical suspension aerosol formulations for use in metered dose inhalers.

Pharmaceutical aerosol formulations typically comprise a drug substance suspended or dissolved in a liquefied gas propellant. A metered dose inhaler typically consists of a canister for storing an aerosol formulation under pressure, which is sealed at one end with a metering valve. The canister and valve are held in an actuator, which comprises means for actuating the metering valve through which may be dispensed a precisely fixed dose of the aerosol formulation, and a mouth-piece through which a dispensed dose of the aerosol formulation is directed into the mouth of a patient.

Each dose of drug released from a metered dose inhaler should be metered with very narrow tolerance of dose variance set by regulatory authorities. Furthermore, as metered dose inhalers usually contain multiple doses of a drug substance, accuracy in dosing must be assured throughout the life of the inhaler, which may be up to one month or even longer.

When a metered dose inhaler contains a suspension aerosol formulation, i.e. an aerosol formulation in which particles of drug substance are suspended in a liquefied gas propellant, it is often a problem that the particles tend to deposit on the inner surfaces of the canister and metering valve. This can result in unacceptable variance in the delivered dose and reduction in delivered dose. Deposition can be a particular problem when a drug substance is used in very low concentrations. If the concentration of drug substance delivered per actuation is very low, then any loss of dose because of deposition could result in serious dosing problems. Formoterol fumarate dihydrate particularly, but also fluticasone propionate are potent materials and are consequently intended for use in very low concentrations.

The prior art has addressed the problem of particle deposition. WO96/32099 discloses a canister that is coated with certain non-stick polymers, such as fluorocarbons, see also for example WO-A-96/32150 and US-A-6,596,260. Draw backs with such polymers include the risk of partial solubility of some of their constituents in some aerosol propellants, and that such coatings themselves need to undergo safety tests and product formulation development in order to ensure a safe and stable product. These polymers and the testing required can add to production costs.

WO 02/24552 discloses a method of applying multiple layers of fluoro polymers to canisters with heating. Not only is this laborious and costly, but problems can arise with regard to certain metals used in the production of canisters. The most commonly used metals for forming canisters are aluminium alloys. The polymer coatings must undergo heat treatment in order to be cured, which can result in the strength of canisters being compromised because the metal can become softer and malleable from the application of heat.

The polymer coating materials themselves can also be incompatible with some components of certain aerosol formulations and can lead to contamination because there is the potential for leachable compounds to find their way into formulations. Such leachable compounds can lead to degradation of drug substances and also result in less effective and less robust products.

WO 2004/019985 uses a surface active agent such as oleic acid to stabilise a triple combination formulation. WO 02/078671 relates to a formulation that includes a solid excipient comprising a salt of palmitic or stearic acid. WO 2005/034911 relates to a formulation in which formoterol is dried before it is added to the other ingredients of the formulation. US 2002/025299 relates to a solution formulation.

In the field of suspension aerosol formulations there remains a need to reduce or eliminate drug particle deposition without the need to resort to the use of coated canisters.

Surprisingly the applicant has found that a wetting agent employed in a suspension aerosol formulation can act to reduce drug particle deposition and improve delivered dose uniformity and the aerodynamic particle size distribution of the delivered dose. This effect is observed with canisters having no surface coating or coatings.

In one aspect of the invention there is provided the use of a wetting agent in a suspension aerosol formulation comprising particles of formoterol fumarate dihydrate and fluticasone propionate suspended in HFA propellant contained in a metered dose inhaler comprising a canister and metering valve, to reduce deposition of particles on the surfaces of the canister and the metering valve, wherein the wetting agent is selected from the group consisting of ethanol, propylene glycol, glycerol, butandiol and mixtures thereof. In a particularly preferred embodiment, the wetting agent is ethanol, and more particularly dehydrated ethanol.

The skilled person will be able to optimise the amount of wetting agent employed having regard to the concentration of the drug substances employed, the nature of the propellant and also the nature of any other adjuvants or excipients employed in the suspension aerosol formulation. If a wetting agent is a solvent for one or both of the drug substances, the wetting agent should be employed in an amount that avoids solubilisation or partial solubilisation of the drug substances or any excipients intended to be held in suspension.

The wetting agent is thought to facilitate the wetting of the particles of the drug substance suspended in the liquefied propellant preventing the drug substance from becoming partially solubilised. Partial solubilisation of a drug substance can cause Ostwald ripening, particle growth, and eventually formulation stability failure. Partial solubilisation would be particularly detrimental in formulations employing drug substances in very low concentrations, as the loss of even the slightest amount of drug substance to these effects would have an exaggerated effect in terms of variance of the dose emitted from an inhaler.

The balance a formulator needs to achieve in providing sufficient wetting agent to achieved a desired effect whilst at the same time avoiding solubilisation can be particularly fine when one employs high potency materials such as formoterol fumarate dihydrate and fluticasone propionate in low or very low concentrations. The balance is made yet more complicated by the presence of two drug substances each having their unique physical properties, such as solubility.

The wetting agent may be employed in an amount of less than 2% by weight, more particularly 1.99 to 0.01% by weight, still more particularly 1.5 to 0.01 % by weight, still more particularly 1.5% to 1.0% by weight based on the total weight of the suspension aerosol formulation.

When a metered dose inhaler is actuated, propellant containing suspended drug substance is expelled under pressure from a metering valve on the metered dose inhaler. The propellant boils off as it leaves the valve and the drug substance particles are released as a stream of micro-fine powder into the oral cavity of a patient. In order that the formulation can be inhaled deep into the lungs to exert a therapeutic effect, the particles of the aerosol formulation need to be micro-fine with a mean aerodynamic particle diameter (measured as Mass Median Aerodynamic Diameter (MMAD)) of about 1 to 10µm, and preferably 1 to 6µm. Micronised particles of this size can be obtained by various methods known in the art, for example mechanical grinding or spray drying. In a preferred method, size reduction of drug particles is carried out by an air jet mill.

The quantity of drug substance delivered in fine, inhalable particles having a MMAD in the range stated above and which is considered able to penetrate and deposit in the lung is known as the fine particle dose (FPD), or expressed as a portion of the delivered dose, the fine particle fraction (FPF). Both of these parameters can be calculated from the measurement of the aerodynamic particle size distribution of a delivered dose with a cascade impactor or liquid impinger. These are routine tests for which the methods and apparatus are described in the pharmacopoeia, see for example Chapter <601> of the United States Pharmacopoeia (USP) 32 or in the inhalants monograph 2.9.18 of the European Pharmacopoeia (Ph.Eur.), 6th edition 2009.

Whereas the micro-fine nature of the drug particles is desirable and necessary for them to penetrate deep into the lung and exert a therapeutic effect, fine particles, owing to their large surface area and therefore an unfavourable ratio of surface area to volume or mass, can exhibit adhesive forces, which can cause particles to deposit on the surfaces of the canister and metering valve surfaces.

The use of a wetting agent in accordance with the present invention minimises these adhesive interactions and reduces the amount of deposition ensuring acceptable delivered dose uniformity and an aerodynamic particle size distribution of the delivered dose that translates to a high fine particle dose and fine particle fraction.

Deposition of particles inside a canister and valve can be measured by assaying the drug substances remaining in exhausted inhalers.

Delivered dose uniformity and aerodynamic particle size distribution may be measured at initial testing. Initial testing as used herein refers to a test of filled canisters at time t= 0 after an initial equilibration storage condition of at least 2 weeks and at ambient temperature and humidity, e.g. about 17 to 25 degrees centigrade and a relative humidity of about 29 to 63%. Testing proceeds with the repeated actuation of an equilibrated canister to exhaustion and the delivered dose, its variance and the aerodynamic particle size distribution is measured by techniques known in the art. Testing a product at "initial" is an important benchmark for formulators. If a product fails to meet acceptable levels for variance of delivered dose or aerodynamic particle size distribution at this stage, then it is an indication that a formulation would fail under conditions of accelerated storage, which is predictive of the performance of a formulation under normal storage and usage conditions e.g. for 2 years or longer at a temperature of 25 degrees centigrade and a relative humidity of 60 % to 75 % or preferably at 30 degrees centigrade and a relative humidity of about 65 %.

Assaying the amount of drug substance deposited on the canister and valve at initial testing is an important aspect of aerosol formulation development. Drug loss is observed to some extent in all parts of manufacturing and application before the drug reaches the patient's lung. There is usually some loss in the manufacturing vessel, pipes and filling line. Furthermore, during storage, one can expect to observe a certain amount of deposition in the canister and closure system. Still further, during application or actuation there is an amount of drug retained on the valve and on the actuator. All this is taken into consideration when determining how much drug should be filled into a canister to achieve the desired dose to be delivered to the patient.

As there are a multitude of deposition sites for drug substance throughout manufacture, filling, storage and application, one must ensure tight control during all aspects of manufacture and storage of a formulation. A very important control is the assay for deposited drug within the canister and closure system (including the valve) at "initial". If the deposition is too high at this stage of the process, then there is no realistic possibility that the formulation will be delivered to a patient with the correct delivered dose, or fine particle fraction.

In the assay for deposition of drug substance, it is important that for both formoterol fumarate dihydrate and for fluticasone propionate, the extent of deposition is below about 12% of the nominal content of the canister, more particularly deposition should be within the range of about 4 to 10% of the nominal content. By "nominal content" is meant, the amount of drug that is weighed into the batch manufacturing vessel divided by number of canisters to be filled with the given amount of bulk suspension formulation.

Accordingly, when the suspension aerosol formulation of the invention is stored in a metered dose inhaler for at least two weeks at a temperature of about 17 to about 25 degrees centigrade and a relative humidity of about 29 to 63%, it preferably contains a residue of formoterol fumarate dihydrate of not more than about 12%, more particularly in a range of about 4 to 10% based on the amount of formoterol fumarate dihydrate filled into the canister.

When the suspension aerosol formulation of the invention is stored in a metered dose inhaler for at least two weeks at a temperature of about 17 to about 25 degrees centigrade and a relative humidity of about 29 to 63%, it preferably contains a residue of fluticasone propionate of not more than about 12%, more particularly in a range of about 4 to 10% based on the amount fluticasone propionate filled into the canister.

The amount of deposition is remarkably low considering that these values can be achieved using canisters whose internal surfaces are not treated with non-stick substances such as fluoropolymers. Canisters for use in the present invention in fact can be of standard aluminium or aluminium alloy, typically of cylindrical cross section that are commonly used in drug aerosol applications. In the assay described above, a suitable canister is sealed by crimping with a suitable metering valve, which valves are commonly known in the art.

It is also desirable that the suspended drug substance particles have a particle size distribution such that D10 (10 % of the volume distribution) is in the range of 0.2 to 2 micro-metres, D50 in the range of 1 to 4 micro-metres and D90 in the range of 2 to 6 micro-metres when measured by laser diffraction either in suspension or as a dispersed dry powder.

The invention provides in another of its aspects a suspension aerosol formulation comprising particles of formoterol fumarate dihydrate and fluticasone propionate suspended in an HFA propellant, and a wetting agent, wherein the wetting agent is selected from the group consisting of ethanol, propylene glycol, butandiol and mixtures thereof and is employed in an amount of less than 2% by weight based on the total weight of the formulation and wherein the particles have a particle size distribution wherein D10 (10 % of the volume distribution) is in the range of 0.2 to 2 micro-metres, D50 in the range of 1 to 4 micro-metres and D90 in the range of 2 to 6 micro-metres when measured by laser diffraction either in suspension or as a dispersed dry powder.

Still more particularly, D10 is about 0.2-1.8, D50 is about 1.0-3.0 and D90 is about 2.0-6.0 micro-metres for formoterol fumarate dihydrate, and for fluticasone propionate D10 is about 0.2-1.8, D50 is about 1.2-3.5 and D90 is about 2.0-6.0 micro-metres.

The delivered dose uniformity and aerodynamic particle size distribution of the delivered dose are important parameters that determine the therapeutic effectiveness of formulations from metered dose inhalers. These parameters can be measured in vitro. The delivered dose (sometimes referred to as the "emitted dose") is the amount of drug substance that is substantially released from an actuator mouthpiece and available for inhalation by a patient. The delivered dose can be different to the dose actually dispensed from an inhaler by the metering valve upon actuation (often referred to as the metered dose). This is because a tiny amount of the metered dose can be deposited on the valve stem and the actuator of the metered dose inhaler.

Delivered dose uniformity is a measure of the variance of the delivered dose and can be used to test the reproducibility of dosing from batch to batch, or for a particular metered dose inhaler, the variability from actuation to actuation through life of the inhaler. Some variance in delivered dose is to be expected, but it must fall within limits proscribed by regulatory authorities if a product is to gain market authorisation.

The aerodynamic particle size distribution informs the formulator of the amount of drug substance particles contained in the delivered dose that are of small enough aerodynamic diameter in order to reach the deep lung upon inhalation, and the fine particle dose and fine particle fraction can be derived from this measurement.

The variance of the delivered dose (the delivered dose uniformity) can be measured using a Dosage Unit Sampling Apparatus (DUSA). The FPF can be determined from measurements of particle size distribution by a cascade impactor or a liquid impinger, for example an Andersen Cascade Impactor (ACI). The measurement methodology and the apparatus employed are well known in the art, and are described in the United States Pharmacopoeia Chapter < 601 > , or in the inhalants monograph of the European Pharmacopoeia. The USP suggests that the Apparatus 1 can be used for the measurement of FPF. The USP also suggests that delivered dose uniformity can be measured with DUSA or its equivalent. Alternatively, however, the delivered dose and delivered dose uniformity may be measured using the Funnel

Method, which is well known in the art.

The Funnel Method is described in Drug Delivery to the Lungs, VIII p116 to 119. In summary, the Funnel Method consists of discharging a formulation from a metered dose inhaler into a Funnel Apparatus, which basically consists of a standard Buchner Funnel. The discharged dose is captured on a glass sinter located within the Funnel, and can be washed off, and the dose is determined using HPLC analysis. The Funnel Method gives comparable results to the standard USP apparatus, and is generally considered to be an equivalent of the DUSA apparatus.

Accordingly, when the formulation of the invention is stored in a metered dose inhaler for at least 2 weeks at about 17 to 25 degrees centigrade and a relative humidity of about 29 to 63%, it preferably has a mean delivered dose through life of the inhaler of about 40 to 500 micrograms fluticasone propionate and 4 to 20 micrograms formoterol fumarate dihydrate.

When the formulation of the invention is stored in a metered dose inhaler for at least 2 weeks at about 17 to 25 degrees centigrade and a relative humidity of about 29 to 63% , it preferably has a mean fine particle fraction of 30 to 60 % of the labelled dose.

In preferred suspension aerosol formulations described herein the particles of formoterol fumarate dihydrate and of fluticasone propionate display a particle size distribution wherein D10 (10 % of the volume distribution) is in the range of 0.2 to 2 micro-metres, D50 in the range of 1 to 4 micro-metres and D90 in the range of 2 to 6 micro-metres when measured by laser diffraction either in suspension or as a dispersed dry powder.

Still more particularly, D10 is about 0.2-1.8, D50 is about 1.0-3.0 and D90 is about 2.0-6.0 micro-metres for formoterol fumarate dihydrate, and for fluticasone propionate D10 is about 0.2-1.8, D50 is about 1.2-3.5 and D90 is about 2.0-6.0 micro-metres.

In preferred suspension aerosol formulations described herein the wetting agent is employed in an amount of less than 2% by weight, more particularly 1.99 to 0.01% by weight, still more particularly 1.5 to 0.01 % by weight, still more particularly 1.5% to 1.0% by weight based on the total weight of the suspension aerosol formulation.

In preferred suspension aerosol formulations described herein formoterol fumarate dihydrate may be employed in an amount of 0.003-0.04% by weight; preferably 0.004-0.03% by weight; and more preferably 0.005-0.02% by weight, based on the total weight of the formulation. In a preferred embodiment, formoterol fumarate dihydrate may be employed in an amount of 0.003-0.008% by weight, based on the total weight of the formulation. In an alternative preferred embodiment, formoterol fumarate dihydrate may be employed in an amount of 0.01 to 0.04% by weight, based on the total weight of the formulation.

In preferred suspension aerosol formulations described herein fluticasone propionate may be present in an amount of 0.01-0.6% by weight; preferably between 0.02 -0.5% by weight; and more preferably 0.03-0.4 % by weight, based on the total weight of the formulation.

Formoterol fumarate dihydrate and fluticasone propionate can be employed in suspension aerosol formulations in varying doses in order that physicians can have flexibility in the manner in which they treat patients. Nominal doses of formoterol fumarate dihydrate may range from about 5 to about 20 micrograms, whereas nominal doses of fluticasone propionate may range from about 50 to about 500 micrograms. The term "nominal dose" is essentially a target dose for a drug substance contained in a metered dose inhaler. Metered dose inhalers of the present invention typically will contain a plurality of nominal doses such that a single inhaler may treat patients over several days or weeks depending on the total number of nominal doses loaded into a canister. The actual dose metered from a metered dose inhaler and the delivered dose are expected to be slightly lower than the nominal dose, but within strictly regulated limits.

Examples of doses in metered dose inhalers according to the present invention are set forth in table 1:

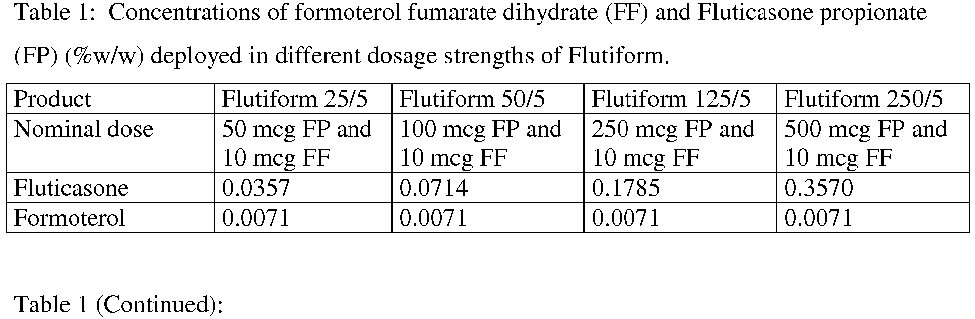

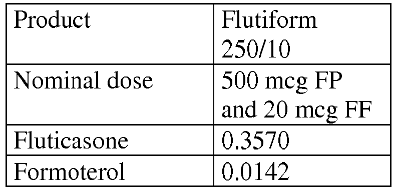

In table 1, the term "Flutiform" relates to the formulation in clinical development by Skyepharma AG. "mcg" is an abbreviation of "micrograms".

The Flutiform product is under development in multiple dosage strengths as shown in the table 1. The Flutiform product 25/5 for example, represents a formulation that dispenses 25 mcg Fluticasone propionate and 5 mcg formoterol fumarate dihydrate per actuation. The inhaler is actuated twice to give the required delivered dose, hence Flutiform 25/5 actually dispenses a dose of 50 mcg Fluticasone propionate and 10 mcg formoterol fumarate dihydrate.

In addition to the components referred to hereinabove, suspension aerosol formulations of the present invention may contain other excipients, which may assist in the manufacture, stabilisation, ease of administration of the formulation, or which are deemed useful or desirable in any other way.

Surfactants may be employed if desired. Surfactants include oleic acid, lecithin, sorbitan trioleate, cetylpyridinium chloride, benzalkonium chloride, polyoxyethylene (20) sorbitan monolaurate, polyoxyethylene (20) sorbitan monostearate, polyoxyethylene (20) sorbitan monooleate, polyoxypropylene/polyoxyethylene block copolymers, polyoxypropylene/polyoxyethylene/ethylenediamine block copolymers, ethoxylated castor oil and the like, where the proportion of surface-active agents, if present, can preferably be approximately 0.0001 to 1% by weight, in particular approximately 0.001 to 0.1% by weight, based on the total formulation.

Furthermore, if desired, the aerosol formulations according to the invention can contain buffer substances or stabilizers such as citric acid, ascorbic acid, sodium EDTA, vitamin E, N-acetylcysteine and the like. In general, such substances, if present, are used in amounts of not more than approximately 1% by weight, for example in an amount of from approximately 0.0001 to 1% by weight, based on the total formulation.

Formulations according to the invention may contain a carrier material. The salts of nedocromil or cromoglycic acid, such as sodium cromoglycate (sometimes referred to as DSCG), are both therapeutically active substances, but their use as moisture-scavenging agents at sub-therapeutic levels has been described in the literature.

The salts of nedocromil or cromoglycic acid, and particularly DSCG may be employed in an amount of 0.01-0.1% by weight, preferably 0.016-0.09% by weight, more preferably 0.02-0.08% by weight, more preferably 0.025-0.07%, more preferably 0.03-0.05% and more preferably 0.03-0.04% by weight, based on the total weight of the formulation.

DSCG has been used for administration via the inhalation route and has been demonstrated to be clinically safe. For providing maximum absorption capacity for water molecules that may enter a metered dose inhaler, the specific surface area of DSCG is preferably increased by reducing the particle size by air jet milling. The preferred moisture scavenging material has a D10 in the range of about 0.2 to 2 micro-metres, D50 in the range of about 1 to 4 micro-metres and D90 in the range of about 2 to 6 micro-metres when measured by a laser diffraction instrument either in suspension or as a dispersed dry powder. More particularly, D10 is not more than 1 micro-metre, D50 is not more than 3 micro-metres and D90 is not more than 5 micro-metres.

It is believed that DSCG acts to aid stabilisation of the aerosol formulations, particularly against hydrolysis by competitive water absorption. DSCG exists as a single crystal form that is non-stoichiometric with regard to water content and adsorbs or desorbs water rapidly in response to changes in relative humidity. DSCG crystals are universal in the extent of reversible water absorption without collapse of the crystal lattice and can absorb up to 9 molecules of water per mol, which is about 24 % w/w. The crystal structure analysis by X-ray diffraction reveals the existence of channels that are capable of reversibly accommodating a variable number of water molecules (depending on the ambient relative humidity) with only small dimensional changes within the lattice. Despite its large moisture adsorption capacity DSCG is not deliquescent (like, for example, sodium sulphate) but is solid in the range of 10 to 90 % r.h..

It is believed that DSCG acts to stabilize the formulation. In particular, it is believed to have a beneficial effect on fine particle fraction (FPF) by competitively binding free (i.e. molecular dissolved) water present within the propellant phase. This assists in stabilising the fine particle fraction by preventing agglomeration of suspended particles (i.e. formation of liquid and/or crystal bridges) and particle growth (i.e. Ostwald ripening) on stability. This allows for a more robust product during storage and use as the formulation has improved tolerance to the presence of internal water. For example, up to 600ppm of total internal water may be tolerated. Furthermore this allows for a much longer 'use period' once the product is in the patients hands.

The moisture-scavenging properties are particularly important when one employs bronchodilator β₂-agonists, such as formoterol fumarate dihydrate owing to their susceptibility to oxidative and hydrolytic conditions. Hydrolysis is one of the major identified factors affecting degradation of formoterol fumarate dihydrate under stress conditions (e.g. 40°C/ 75% relative humidity) because such formulations are usually sensitive to moisture and is susceptible to the ingress of moisture from the surrounding air.

Slight concentration changes or changes in the physical stability of suspension aerosol formulations, which may occur during storage due to temperature changes and/or moisture ingress may lead to significant reductions or variance in the delivered dose and fine particle dose.

DSCG also acts as a suspension enabling agent when used in aerosol formulations of the present invention. DSCG itself consists of particles which encourage and allow the formation of heterogeneous floccules with particles, and this is believed to produce better suspensions with lower tendency to cream or to sediment. This in turn leads to improvements in the stability and robustness of the formulations of the present invention.

DSCG therefore is an advantageous excipient to ensure through-life robustness and stability to aerosol formulations of the present invention. Furthermore, having regard to the foregoing, aerosol formulations containing a wetting agent and DSCG in the amounts referred to herein are particularly preferred formulations as the wetting agent ensures good dose uniformity at initial testing and the DSCG ensures that the performance in this parameter and others is stable and robust through life of the container.

The invention therefore provides in yet another aspect, in a suspension aerosol formulation as hereinabove described, the use of a wetting agent and DSCG in the amounts referred to hereinabove to provide dose uniformity throughout the life of the formulation.

Examples of particularly preferred dosage strengths of suspension aerosol formulations useful in the present invention may be found in the following table.

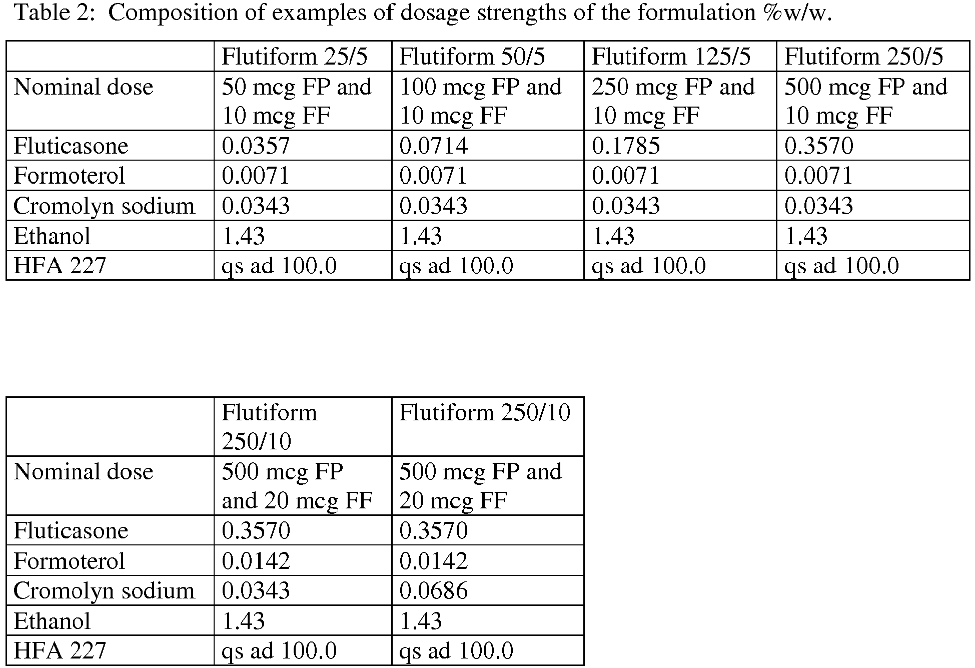

The suspension aerosol formulations described herein can be loaded into metered dose inhalers and deliver consistent doses of the drug substances through the life of the inhalers. The formulations of the present invention meet compendial requirements regarding delivered dose uniformity as set forth, by way of example, the United States and European pharmacopoeias. More particularly, the formulations of the present invention meet the requirements as set out in the USP26-NF21 chapter 601 "dose uniformity".

Accordingly, the invention provides in another of its aspects a suspension aerosol formulation as hereinabove described, wherein the formulation, when dispensed from a metered dose inhaler delivers a dose of both the formoterol fumarate dihydrate and fluticasone propionate that has a variance of no more that +/- 15% of the target mean dose (and not more than 1 value outside +/- 25% of target and none outside +/- 30% of target) when the formulation is stored at 25 degrees centigrade and 60% relative humidity, more particularly 40 degrees centigrade and 75% relative humidity for a period of up to 1 month, more particularly up to 3 months, still more particularly up to 6 months.

There is provided in another aspect of the invention a suspension aerosol formulation as described herein for use in the treatment of asthma, chronic obstructive pulmonary disease (COPD) or allergic rhinitis.

A suspension aerosol formulation comprising formoterol fumarate dihydrate and fluticasone propionate as drug substances is intended as a therapy for controlling the symptoms of asthma and COPD. Such formulations, for this reason, can be referred to as controller medications. An adequately controlled patient should not require any other therapy for the treatment of asthma or COPD symptoms. However, it is possible that between receiving doses of the suspension aerosol formulation a patient may experience an exacerbation of symptoms, in which case the patient may receive a dose of a short-acting beta 2 agonist. Examples of such beta-2- agonists include albuterol, salbutamol, terbutaline, fenoterol, levalbuterol, reproterol and pirbuterol.

In another aspect the invention provides a suspension aerosol formulation as described herein for use in the treatment of asthma or chronic obstructive pulmonary disease (COPD), simultaneously, sequentially or separately with a short-acting beta-2-agonist selected from the group consisting of albuterol, salbutamol, terbutaline, fenoterol, levalbuterol, reproterol and pirbuterol.

Patients receiving or indicated to receive a controller medication comprising a suspension aerosol formulation comprising formoterol fumarate dihydrate and fluticasone propionate may be receiving alternative controller medications or no medications at all. A typical controller medication may be an inhaled corticosteroid. Typical inhaled corticosteroids include beclamethasone dipropionate, budesonide, ciclesonide, flunisolide, fluticasone propionate, mometasone furoate and triamcinolone acetonide. The dosages of these materials that a patient may be receiving will depend on the particular steroid employed.

The suspension aerosol formulation may comprise a dose of 10 to 20 micrograms formoterol fumarate dihydrate and 50 to 100 micrograms fluticasone propionate BID, or 10 micrograms formoterol fumarate dihydrate and 250 to 500 micrograms fluticasone propionate BID depending on the whether the patient is receiving low, medium or high dose inhaled corticosteroid.

In the event of asthma or COPD deteriorating over time, the doses of formoterol fumarate dihydrate and fluticasone propionate employed in the suspension aerosol formulations may be increased or additional controller medications may be added to a patient's therapy. Additional therapies may include leukotriene modifiers or sustained release methylxanthine formulations.

In the methods of the present invention, the suspension aerosol formulations containing formoterol fumarate dihydrate and fluticasone propionate are preferably those described hereinabove.

The suspension aerosol formulations describe for use in the treatments describe above are intended as BID, that is, twice daily administration on a regular basis as part of a therapy for controlling the symptoms of asthma and COPD. Physicians will assess the condition of patients receiving such treatment and adjust the dosage according to need.

Each dosage administration may be delivered with a single actuation of a metered dose inhaler, or the dose may be administered in two or more actuations. Preferably, each dose is delivered in two actuations of a metered dose inhaler.

There now follows a series of examples that serve to illustrate the invention

### Example 1

The following compositions shown below in Table 3 were prepared.

**Table 3: Compositions of pharmaceutical formulations.**

| | Flutiform 25/5 | Flutiform 50/5 | Flutiform 125/5 | Flutiform 250/5 | Flutiform 250/10 |
|---|---|---|---|---|---|
| Nominal dose | 50 mcg FP and 10 mcg FF | 100 mcg FP and 10 mcg FF | 250 mcg FP and 10 mcg FF | 500 mcg FP and 10 mcg FF | 500 mcg FP and 20 mcg FF |
| Fluticasone | 0.0357 | 0.0714 | 0.1785 | 0.3570 | 0.3570 |
| Formoterol | 0.0071 | 0.0071 | 0.0071 | 0.0071 | 0.0142 |
| Cromolyn sodium | 0.0343 | 0.0343 | 0.0343 | 0.0343 | 0.0343 |
| Ethanol | 1.43 | 1.43 | 1.43 | 1.43 | 1.43 |
| HFA 227 | qs ad 100.0 | qs ad 100.0 | qs ad 100.0 | qs ad 100.0 | qs ad 100.0 |

The appropriate amounts of the micronised active substances were weighed and transferred into the batching vessel. The appropriate amount of micronised sodium cromolyn, (DSCG) was added and the vessel closed. The propellant mixture of HFA 227 (apaflurane) with 1.43 % alcohol was made in a separate vessel and transferred into the batching vessel. The solid materials were dispersed in the liquefied propellant by use of a rotor-stator homogenizer at 2900 rpm for 30 min. The homogeneous bulk suspension was cooled to 4 °C and re-circulated between the vessel and the Pamasol aerosol filling machine P2001.

Pharmaceutical aerosol canisters with 14 ml brim full volume were crimped with 50 mcl metering valves using a Pamasol P2005 crimping machine. Aliquots of 11 g suspension were filled into the crimped canisters by the P2001 filling machine. The weight of each filled canister was checked; all filled canisters were subjected to a heat stress test at about 50 to 60 °C and stored one month prior to assembly with an actuator for testing.

### Example 2

The Flutiform 50/5 composition shown in Table 2 above, was tested against a comparator product identical to the 50/5 formulation but for the ethanol being removed from the comparator product.

MDI canisters filled with these compositions were tested for assay of both drugs in the canister, dose uniformity through inhaler life up to the last nominal dose, aerodynamic particle size distribution of the aerosol medication by Andersen Cascade Impactor, and for drug deposition on the internal surfaces of the canister and valve (CCS).

The results are shown in Table 3:

**Table 3: Effect of Ethanol on dose uniformity and Aerodynamic Particle Size Distribution (APSD). Results at initial.**

| | Flutiform 50/5 | Comparator |
|---|---|---|
| **Total drug content per container** | | |
| FP assay % of target (RSD[%}); n=3 | 95.1(0.4) | 95.4(1.0) |
| FF assay % of target (RSD[% }); n=3 | 95.5 (0.4) | 100.8 (1.1) |

| **dose uniformity through container life (intra inhaler)** | | |
|---|---|---|
| Mean FP dose µg/actuation (RSD[%]); n=9 | 45 (8.0) | 31 (13.7) |
| Mean FF dose µg/actuation (RSD[%]); n=9 | 4.4 (7.7) | 3.5 (13.5) |

| **APSD by Anderson cascade impactor** | | |
|---|---|---|
| Mean metered FP dose µg/actuation (n=5) | 44.9 | 34.2 |
| Mean FP dose µg/actuation (n=5) | 42.5 | 30.3 |
| Mean fine particle dose FP µg/actuation (n=5) | 16.7 | 12.5 |
| Mean metered FF dose µg/actuation (n=5) | 4.6 | 4.4 |
| Mean FF dose µg/actuation (n=5) | 4.2 | 3.4 |
| Mean fine particle dose FF µg/actuation (n=5) | 2.13 | 1.55 |

| **Residue in CCS*** | | |
|---|---|---|
| FP assay % of target (RSD[%}); n=3 | 7.8 (13.6) | 14.5 (12.8) |
| FF assay % of target (RSD[% }); n=3 | 8.1 (13.8) | 14.7 (14.1) |
| Cromolyn sodium assay % of target (RSD[%}); n=3 | 7.7 (15.1) | 15.9 (13.3) |

The purpose of this experiment was to test the effect of a wetting agent, in this case dehydrated ethanol, on critical parameters such as dose uniformity and particle size distribution. The results show that at initial (results at t= 0, after at least 2 weeks storage at ambient conditions of 17 to 25 degrees centigrade and 29 to 63% relative humidity for the formulation without ethanol, the mean dose and fine particle dose was reduced. Furthermore, the assay of residual material in exhausted containers indicated a marked increase in deposition on internal surfaces of the container closure system for the formulation not containing ethanol.

Testing a product at initial is an important benchmark for formulators. If a product fails in key parameters such as dose and aerodynamic particle size distribution at this stage, it is extremely unlikely that the product will be stable under conditions of accelerated storage.

### Example 3.

The following batches were made up using the process described in Example 1:

**Table 14:**

| Description | Fluticasone/formoterol formulation (nominal dose 500 µg fluticasone /20 µg formoterol) | Fluticasone/formoterol formulation (nominal dose 500 µg fluticasone /10 µg formoterol) |
|---|---|---|
| Composition | % w/w | % w/w |
| Fluticasone propionate | 0.3571 | 0.3571 |
| Formoterol fumarate dihydrate | 0.0143 | 0.0071 |
| DSCG | 0.0343 | 0.0343 |
| Ethanol | 1.43 | 1.43 |
| HFA 227 | qs to 100.0 | qs to 100.0 |

The results of the stability investigation up to 12 months demonstrated good product quality and robustness of both formulations, as shown by the results displayed in Tables 15 and 16, below.

**Table 15: Summary of ACI results of fluticasone/formoterol formulation Flutiform 250/10 up to 12 months at 25 °C / 60 %RH and 40 °C / 75 %RH. Each result is the mean of 6 determinations (beginning and end of 3 canisters).**

| Fluticasone | 25 °C / 60 %RH | | | | | | | 40 °C / 75 %RH | | | | | | |
|---|---|---|---|---|---|---|---|---|---|---|---|---|---|---|
| | Initial | 3 months | | 6 months | | 12 months | | 1 month | | 3 months | | 6 months | | 12 months |
| Metered dose [µg] | 428.9 | 419.8 | 426.8 | 427.7 | 425.9 | 454.8 | 429.3 | 437.1 | 412.6 | 442.3 | 449.8 | 430.0 | 431.2 | 447.9 |
| Delivered dose [µg] | 407.5 | 400.9 | 407.3 | 413.9 | 408.5 | 434.2 | 413.5 | 417.0 | 392.2 | 423.2 | 417.3 | 415.6 | 414.1 | 429.8 |
| FPD (Stage 3 - Filter) [µg] | 173.6 | 184.0 | 179.1 | 181.8 | 184.0 | 186.1 | 177.0 | 180.1 | 177.0 | 193.2 | 183.1 | 174.7 | 181.0 | 172.1 |
| | | | | | | | | | | | | | | |
| Group 1 | | | | | | | | | | | | | | |
| (Mouthpiece - USP throat) [µg] | 186.2 | 172.6 | 185.2 | 179.5 | 180.3 | 195.9 | 182.6 | 185.6 | 164.9 | 179.9 | 206.5 | 182.6 | 179.6 | 196.6 |
| Group 2 (Stage 0 - Stage 2) [µg] | 60.2 | 52.3 | 52.6 | 56.7 | 53.5 | 65.0 | 60.8 | 60.7 | 61.3 | 59.7 | 52.5 | 63.3 | 61.5 | 69.9 |
| Group 3 (Stage 3 - Stage5) [µg] | 168.8 | 178.5 | 174.2 | 177.1 | 179.2 | 181.1 | 172.1 | 175.1 | 172.4 | 188.6 | 178.1 | 170.7 | 176.6 | 168.5 |
| Group 4 (Stage 6 - Filter) [µg] | 4.9 | 5.5 | 5.0 | 4.7 | 4.8 | 5.0 | 4.8 | 5.0 | 4.6 | 4.7 | 5.0 | 3.9 | 4.4 | 3.7 |
| | | | | | | | | | | | | | | |
| Formoterol | | | | | | | | | | | | | | |
| Metered dose [µg] | 18.50 | 18.27 | 18.12 | 18.22 | 18.00 | 19.25 | 17.60 | 18.15 | 17.51 | 18.59 | 19.05 | 18.42 | 18.21 | 18.43 |
| Delivered dose [µg] | 16.89 | 16.71 | 16.47 | 16.89 | 16.57 | 17.54 | 16.34 | 16.76 | 16.05 | 17.13 | 16.56 | 17.04 | 16.76 | 16.81 |
| FPD (Stage 3 - Filter) [µg] | 8.40 | 8.87 | 8.66 | 8.90 | 9.04 | 9.01 | 8.29 | 8.61 | 8.48 | 9.23 | 8.92 | 8.64 | 9.00 | 8.23 |
| | | | | | | | | | | | | | | |
| Group 1 | | | | | | | | | | | | | | |
| (Mouthpiece - USP throat) [µg] | 8.06 | 7.61 | 7.67 | 7.42 | 7.21 | 8.16 | 7.30 | 7.49 | 7.01 | 7.42 | 8.44 | 7.72 | 7.23 | 8.03 |
| Group 2 (Stage 0 - Stage 2) [µg] | 1.75 | 1.41 | 1.45 | 1.57 | 1.45 | 1.81 | 1.68 | 1.68 | 1.70 | 1.61 | 1.42 | 1.74 | 1.67 | 1.85 |
| Group 3 (Stage 3 - Stage 5) [µg] | 8.18 | 8.60 | 8.43 | 8.68 | 8.81 | 8.77 | 8.08 | 8.36 | 8.27 | 9.00 | 8.69 | 8.44 | 8.79 | 8.05 |
| Group 4 (Stage 6 - Filter) [µg] | 0.22 | 0.27 | 0.23 | 0.23 | 0.23 | 0.23 | 0.21 | 0.25 | 0.21 | 0.23 | 0.23 | 0.20 | 0.21 | 0.18 |

**Table 16: Summary of ACI results of fluticasone/formoterol formulation Flutiform 250/5 up to 12 months at 25 °C / 60 %RH and 40 °C / 75 %RH. Each result is the mean of 6 determinations (beginning and end of 3 canisters).**

| Fluticasone | 25 °C / 60 %RH | | | | | 40 °C / 75 %RH | | | | |
|---|---|---|---|---|---|---|---|---|---|---|
| | Initial | 6 months | | 12 months | | 1 month | | 6 months | | 12 months |
| Metered dose [µg] | 402.2 | 432.1 | 426.9 | 433.0 | 420.3 | 419.0 | 417.1 | 431.4 | 428.9 | 417.9 |
| Delivered dose [µg] | 378.4 | 413.9 | 411.6 | 416.2 | 405.5 | 404.5 | 401.0 | 412.1 | 417.3 | 402.7 |
| FPD (Stage 3 - Filter) [µg] | 181.0 | 203.2 | 195.1 | 193.6 | 185.6 | 185.0 | 181.2 | 194.1 | 193.9 | 178.9 |
| | | | | | | | | | | |
| Group 1 (Mouthpiece - USP throat) [µg] | 171.5 | 175.9 | 180.1 | 175.6 | 178.9 | 181.4 | 183.7 | 179.9 | 175.5 | 171.2 |
| Group 2 (Stage 0 - Stage 2) [µg] | 42.1 | 46.3 | 46.2 | 54.2 | 47.8 | 43.6 | 45.0 | 50.8 | 52.3 | 58.1 |
| Group 3 (Stage 3 - Stage 5) [µg] | 177.1 | 199.1 | 190.8 | 189.9 | 182.0 | 181.0 | 177.2 | 190.7 | 190.3 | 175.7 |
| Group 4 (Stage 6 - Filter) [µg] | 3.8 | 4.0 | 4.4 | 3.7 | 3.6 | 4.0 | 4.0 | 3.4 | 3.5 | 3.2 |
| | | | | | | | | | | |

| Formoterol | | | | | | | | | | |
|---|---|---|---|---|---|---|---|---|---|---|
| Metered dose [µg] | 8.47 | 9.28 | 9.22 | 9.22 | 8.78 | 9.09 | 9.10 | 9.13 | 9.07 | 8.49 |
| Delivered dose [µg] | 7.62 | 8.38 | 8.40 | 8.45 | 8.03 | 8.36 | 8.26 | 8.23 | 8.37 | 7.76 |
| FPD (Stage 3 - Filter) [µg] | 3.81 | 4.42 | 4.28 | 4.27 | 4.04 | 4.08 | 4.00 | 4.19 | 4.20 | 3.82 |
| | | | | | | | | | | |
| Group 1 (Mouthpiece - USP throat) [µg] | 3.78 | 3.91 | 4.01 | 3.84 | 3.79 | 4.04 | 4.14 | 3.94 | 3.84 | 3.59 |
| Group 2 (Stage 0 - Stage 2) [µg] | 0.75 | 0.82 | 0.81 | 0.92 | 0.81 | 0.79 | 0.81 | 0.86 | 0.89 | 0.92 |
| Group 3 (Stage 3 - Stage 5) [µg] | 3.75 | 4.34 | 4.19 | 4.17 | 3.97 | 3.96 | 3.89 | 4.11 | 4.11 | 3.75 |
| Group 4 (Stage 6 - Filter) [µg] | 0.06 | 0.08 | 0.10 | 0.10 | 0.08 | 0.11 | 0.11 | 0.08 | 0.09 | 0.07 |

## Claims

1. Use of a wetting agent in a suspension aerosol formulation comprising particles of formoterol fumarate dihydrate and fluticasone propionate suspended in HFA propellant contained in a metered dose inhaler comprising a canister and metering valve, to reduce deposition of particles on the surfaces of the canister and metering valve, wherein the wetting agent is selected from the group consisting of ethanol, propylene glycol, glycerol, butandiol and mixtures thereof, preferably wherein the wetting agent is ethanol.

2. The use according to claim 1 wherein the wetting agent is employed in an amount of less than 2% by weight based on the total weight of the suspension aerosol formulation, preferably 1.99 to 0.01% by weight based on the total weight of the suspension aerosol formulation, more preferably 1.5% to 1.0% by weight based on the total weight of the suspension aerosol formulation.

3. The use according to claim 1 wherein the HFA propellant is HFA 227.

4. The use according to claim 1 wherein the formoterol fumarate dihydrate is employed in an amount of 0.003-0.04% by weight.

5. The use according to claim 1 wherein fluticasone propionate is employed in an amount of 0.01-0.6% by weight.

6. The use according to claim 1 wherein the suspension aerosol formulation comprises a salt selected from the group consisting of nedocromil and cromoglycic acid, preferably wherein the salt is sodium cromoglycate (DSCG), preferably wherein the sodium cromoglycate is employed in an amount of 0.01-0.1% by weight based on the total weight of the formulation.

7. The use according to claim 1, wherein the suspension aerosol formulation when dispensed from the metered dose inhaler delivers a dose of both the formoterol fumarate dihydrate and fluticasone propionate that has a variance of no more than +/- 15% of the target mean delivered dose (and not more than 1 value outside +/- 25% of target and none outside +/- 30% of target) when the formulation is stored at 25 degrees centigrade and 60% relative humidity, more particularly 40 degrees centigrade and 75% relative humidity for a period of up to 1 month, more particularly up to 3 months, still more particularly up to 6 months.

8. The use according to claim 1, wherein the suspension aerosol formulation when stored in the metered dose inhaler for at least 2 weeks and a temperature of 17 to 25 degrees centigrade and a relative humidity of 29 to 63% has a mean fine particle fraction of 30 to 60 % of the labelled dose as in-vitro representation of the aerodynamic particle size distribution.

9. The use according to claim 1, wherein the suspension aerosol formulation contained in the metered dose inhaler comprises:
i) 0.035% fluticasone propionate, 0.007% formoterol fumarate, 0.034% sodium cromoglycate, 1.4% ethanol and HFA 227 qs ad 100 wherein all percentages are based on the total weight of the composition, and wherein the nominal dose of fluticasone propionate is 50 micrograms, and for formoterol fumarate 10 micrograms, two times per day (BID);
ii) 0.071% fluticasone propionate, 0.007% formoterol fumarate, 0.034% sodium cromoglycate, 1.4% ethanol and HFA 227 qs ad 100 wherein all percentages are based on the total weight of the composition, and wherein the nominal dose of fluticasone propionate is 100 micrograms and for formoterol fumarate 10 micrograms, two times per day (BID);
iii) 0.178% fluticasone propionate, 0.007% formoterol fumarate, 0.034% sodium cromoglycate, 1.4% ethanol and HFA 227 qs ad 100 wherein all percentages are based on the total weight of the composition, and wherein the nominal dose of fluticasone propionate is 250 micrograms, and for formoterol fumarate 10 micrograms two times per day (BID);
iv) 0.357% fluticasone propionate, 0.007% formoterol fumarate, 0.034% sodium cromoglycate, 1.4% ethanol and HFA 227 qs ad 100 wherein all percentages are based on the total weight of the composition, and wherein the nominal dose of fluticasone propionate is 500 micrograms, and for formoterol fumarate 10 micrograms, two times per day (BID);
v) 0.357% fluticasone propionate, 0.014% formoterol fumarate, 0.068% sodium cromoglycate, 1.4% ethanol and HFA 227 qs ad 100 wherein all percentages are based on the total weight of the composition, and wherein the nominal dose of fluticasone propionate is 500 micrograms and for formoterol fumarate 20 micrograms, two times per day; or
vi) 0.357% fluticasone propionate, 0.014% formoterol fumarate, 0.034% sodium cromoglycate, 1.4% ethanol and HFA 227 qs ad 100 wherein all percentages are based on the total weight of the composition, and wherein the nominal dose of fluticasone propionate is 500 micrograms and for formoterol fumarate 20 micrograms, two times per day.

10. A suspension aerosol formulation comprising particles of formoterol fumarate dihydrate and fluticasone propionate suspended in HFA propellant and a wetting agent wherein the wetting agent is selected from the group consisting of ethanol, propylene glycol, glycerol, butandiol and mixtures thereof and is employed in an amount of less than 2% by weight based on the total weight of the formulation and wherein the particles have a particle size distribution wherein D10 (10% of the volume distribution) is in the range of 0.2 to 2 micro-metres, D50 is in the range of 1 to 4 micrometres and D90 is in the range of 2 to 6 micrometres when measured by laser diffraction either in suspension or as a dispersed dry powder.

11. A suspension aerosol formulation according to claim 10 wherein the wetting agent is employed in an amount of 1.99 to 0.01% by weight based on the total weight of the suspension aerosol formulation, preferably in an amount of 1.5% to 1 % by weight based on the total weight of the suspension aerosol formulation.

12. A suspension aerosol formulation according to claim 10 wherein the HFA propellant is HFA 227.

13. A suspension aerosol formulation according to claim 10 wherein the formoterol fumarate dihydrate is employed in an amount of 0.003-0.04% by weight.

14. A suspension aerosol formulation according to claim 10 wherein fluticasone propionate is employed in an amount of 0.01-0.6% by weight.

15. A suspension aerosol formulation according to claim 10 wherein the suspension aerosol formulation additionally comprises a salt selected from the group consisting of nedocromil and cromoglicic acid, preferably wherein the salt is sodium cromoglycate (DSCG), more preferably wherein the sodium cromoglycate is employed in an amount of 0.01-0.1% by weight based on the total weight of the formulation.

16. A suspension aerosol formulation according to claim 10 contained in a metered dose inhaler comprising:
(i) 0.035% fluticasone propionate, 0.007% formoterol fumarate, 0.034% sodium cromoglycate, 1.4% ethanol and HFA 227 qs ad 100 wherein all percentages are based on the total weight of the composition, and wherein the nominal dose of fluticasone propionate is 50 micrograms, and for formoterol fumarate 10 micrograms, two times per day (BID);
(ii) 0.071% fluticasone propionate, 0.007% formoterol fumarate, 0.034% sodium cromoglycate, 1.4% ethanol and HFA 227 qs ad 100 wherein all percentages are based on the total weight of the composition, and wherein the nominal dose of fluticasone propionate is 100 micrograms and for formoterol fumarate 10 micrograms, two times per day (BID);
(iii) 0.178% fluticasone propionate, 0.007% formoterol fumarate, 0.034% sodium cromoglycate, 1.4% ethanol and HFA 227 qs ad 100 wherein all percentages are based on the total weight of the composition, and wherein the nominal dose of fluticasone propionate is 250 micrograms, and for formoterol fumarate 10 micrograms two times per day (BID);
(iv) 0.357% fluticasone propionate, 0.007% formoterol fumarate, 0.034% sodium cromoglycate, 1.4% ethanol and HFA 227 qs ad 100 wherein all percentages are based on the total weight of the composition, and wherein the nominal dose of fluticasone propionate is 500 micrograms, and for formoterol fumarate 10 micrograms, two times per day (BID);
(v) 0.357% fluticasone propionate, 0.014% formoterol fumarate, 0.068% sodium cromoglycate, 1.4% ethanol and HFA 227 qs ad 100 wherein all percentages are based on the total weight of the composition, and wherein the nominal dose of fluticasone propionate is 500 micrograms and for formoterol fumarate 20 micrograms, two times per day; or
(vi) 0.357% fluticasone propionate, 0.014% formoterol fumarate, 0.034% sodium cromoglycate, 1.4% ethanol and HFA 227 qs ad 100 wherein all percentages are based on the total weight of the composition, and wherein the nominal dose of fluticasone propionate is 500 micrograms and for formoterol fumarate 20 micrograms, two times per day.

17. A suspension aerosol formulation according to claim 10 for use in the treatment of asthma, allergic rhinitis or chronic obstructive pulmonary disease (COPD).

18. A suspension aerosol formulation according to claim 17 for use simultaneously, sequentially or separately with a short-acting beta-2-agonist, wherein preferably the short-acting beta-2-agonist is selected from the group consisting of albuterol, salbutamol, terbutaline, fenoterol, levalbuterol, reproterol and pirbuterol.

## Patentansprüche

1. Verwendung eines Benetzungsmittels in einer Suspensions-Aerosolformulierung, die in HFA-Treibmittel suspendierte Partikel von Formoterolfumarat-Dihydrat und Fluticasonpropionat beinhaltet, die in einem Dosierinhalator enthalten sind, der einen Behälter und ein Dosierventil beinhaltet, zum Reduzieren der Ablagerung von Partikeln auf den Oberflächen des Behälters und Dosierventils, wobei das Benetzungsmittel aus der Gruppe ausgewählt ist, die aus Ethanol, Propylenglykol, Glycerol, Butandiol und Mischungen davon besteht, wobei das Benetzungsmittel bevorzugt Ethanol ist.

2. Verwendung nach Anspruch 1, wobei das Benetzungsmittel in einer Menge von weniger als 2 Gewichts-%, bezogen auf das Gesamtgewicht der Suspensions-Aerosolformulierung, bevorzugt von 1,99 bis 0,01 Gewichts-%, bezogen auf das Gesamtgewicht der Suspensions-Aerosolformulierung, bevorzugter von 1,5 bis 1,0 Gewichts-%, bezogen auf das Gesamtgewicht der Suspensions-Aerosolformulierung, eingesetzt wird.

3. Verwendung nach Anspruch 1, wobei das HFA-Treibmittel HFA 227 ist.

4. Verwendung nach Anspruch 1, wobei das Formoterolfumarat-Dihydrat in einer Menge von 0,003-0,04 Gewichts-% eingesetzt wird.

5. Verwendung nach Anspruch 1, wobei Fluticasonpropionat in einer Menge von 0,01-0,6 Gewichts-% eingesetzt wird.

6. Verwendung nach Anspruch 1, wobei die Suspensions-Aerosolformulierung ein Salz beinhaltet, das aus der Gruppe ausgewählt ist, die aus Nedocromil und Cromoglicinsäure besteht, wobei das Salz bevorzugt Natriumcromoglicat (DSCG) ist, wobei das Natriumcromoglicat bevorzugt in einer Menge von 0,01-0,1 Gewichts-%, bezogen auf das Gesamtgewicht der Formulierung, eingesetzt wird.

7. Verwendung nach Anspruch 1, wobei die Suspensions-Aerosolformulierung, wenn sie aus dem Dosierinhalator abgegeben wird, eine Dosis sowohl des Formoterolfumarat-Dihydrats und Fluticasonpropionats zuführt, die eine Varianz von nicht mehr als +/- 15% der mittleren zugeführten Zieldosis (und nicht mehr als 1 Wert außerhalb +/- 25% des Zielwerts und keinen außerhalb +/-30% des Zielwerts) aufweist, wenn die Formulierung bei 25 Grad Celsius und einer relativen Luftfeuchtigkeit von 60%, insbesondere bei 40 Grad Celsius und einer relativen Luftfeuchtigkeit von 75% für einen Zeitraum von bis zu 1 Monat, insbesondere bis zu 3 Monaten, ganz besonders bis zu 6 Monaten gelagert wird.

8. Verwendung nach Anspruch 1, wobei die Suspensions-Aerosolformulierung, wenn sie in dem Dosierinhalator für mindestens 2 Wochen und bei einer Temperatur von 17 bis 25 Grad Celsius und einer relativen Luftfeuchtigkeit von 29 bis 63% gelagert wird, eine mittlere Feinpartikelfraktion von 30 bis 60% der zulassungskonformen Dosis als In-vitro-Repräsentation der aerodynamischen Partikelgrößenverteilung aufweist.

9. Verwendung nach Anspruch 1, wobei die in dem Dosierinhalator enthaltene Suspensions-Aerosolformulierung Folgendes beinhaltet:
(i) 0,035% Fluticasonpropionat, 0,007% Formoterolfumarat, 0,034% Natriumcromoglicat, 1,4% Ethanol und HFA 227 q.s. ad 100, wobei alle Prozentangaben auf das Gesamtgewicht der Zusammensetzung bezogen sind und wobei die Nominaldosis von Fluticasonpropionat 50 Mikrogramm und für Formoterolfumarat 10 Mikrogramm zweimal täglich (BID) beträgt;
(ii) 0,071% Fluticasonpropionat, 0,007% Formoterolfumarat, 0,034% Natriumcromoglicat, 1,4% Ethanol und HFA 227 q.s. ad 100, wobei alle Prozentangaben auf das Gesamtgewicht der Zusammensetzung bezogen sind und wobei die Nominaldosis von Fluticasonpropionat 100 Mikrogramm und für Formoterolfumarat 10 Mikrogramm zweimal täglich (BID) beträgt;
(iii) 0,178% Fluticasonpropionat, 0,007% Formoterolfumarat, 0,034% Natriumcromoglicat, 1,4% Ethanol und HFA 227 q.s. ad 100, wobei alle Prozentangaben auf das Gesamtgewicht der Zusammensetzung bezogen sind und wobei die Nominaldosis von Fluticasonpropionat 250 Mikrogramm und für Formoterolfumarat 10 Mikrogramm zweimal täglich (BID) beträgt;
(iv) 0,357% Fluticasonpropionat, 0,007% Formoterolfumarat, 0,034% Natriumcromoglicat, 1,4% Ethanol und HFA 227 q.s. ad 100, wobei alle Prozentangaben auf das Gesamtgewicht der Zusammensetzung bezogen sind und wobei die Nominaldosis von Fluticasonpropionat 500 Mikrogramm und für Formoterolfumarat 10 Mikrogramm zweimal täglich (BID) beträgt;
(v) 0,357% Fluticasonpropionat, 0,014% Formoterolfumarat, 0,068% Natriumcromoglicat, 1,4% Ethanol und HFA 227 q.s. ad 100, wobei alle Prozentangaben auf das Gesamtgewicht der Zusammensetzung bezogen sind und wobei die Nominaldosis von Fluticasonpropionat 500 Mikrogramm und für Formoterolfumarat 20 Mikrogramm zweimal täglich beträgt; oder
(vi) 0,357% Fluticasonpropionat, 0,014% Formoterolfumarat, 0,034% Natriumcromoglicat, 1,4% Ethanol und HFA 227 q.s. ad 100, wobei alle Prozentangaben auf das Gesamtgewicht der Zusammensetzung bezogen sind und wobei die Nominaldosis von Fluticasonpropionat 500 Mikrogramm und für Formoterolfumarat 20 Mikrogramm zweimal täglich beträgt.

10. Eine Suspensions-Aerosolformulierung, die Folgendes beinhaltet: Partikel von Formoterolfumarat-Dihydrat und Fluticasonpropionat, die in HFA-Treibmittel suspendiert sind, und ein Benetzungsmittel, wobei das Benetzungsmittel aus der Gruppe ausgewählt ist, die aus Ethanol, Propylenglykol, Glycerol, Butandiol und Mischungen davon besteht und in einer Menge von weniger als 2 Gewichts-%, bezogen auf das Gesamtgewicht der Formulierung, eingesetzt wird und wobei die Partikel eine Partikelgrößenverteilung aufweisen, wobei D10 (10% der Volumenverteilung) im Bereich von 0,2 bis 2 Mikrometer liegt, D50 im Bereich von 1 bis 4 Mikrometer liegt und D90 im Bereich von 2 bis 6 Mikrometer liegt, wenn die Messung durch Laserdiffraktion entweder in Suspension oder als ein dispergiertes trockenes Pulver erfolgt.

11. Suspensions-Aerosolformulierung nach Anspruch 10, wobei das Benetzungsmittel in einer Menge von 1,99 bis 0,01 Gewichts-%, bezogen auf das Gesamtgewicht der Suspensions-Aerosolformulierung, bevorzugt in einer Menge von 1,5 bis 1 Gewichts-%, bezogen auf das Gesamtgewicht der Suspensions-Aerosolformulierung, eingesetzt wird.

12. Suspensions-Aerosolformulierung nach Anspruch 10, wobei das HFA-Treibmittel HFA 227 ist.

13. Suspensions-Aerosolformulierung nach Anspruch 10, wobei das Formoterolfumarat-Dihydrat in einer Menge von 0,003-0,04 Gewichts-% eingesetzt wird.

14. Suspensions-Aerosolformulierung nach Anspruch 10, wobei Fluticasonpropionat in einer Menge von 0,01-0,6 Gewichts-% eingesetzt wird.

15. Suspensions-Aerosolformulierung nach Anspruch 10, wobei die Suspensions-Aerosolformulierung zusätzlich ein Salz beinhaltet, das aus der Gruppe ausgewählt ist, die aus Nedocromil und Cromoglicinsäure besteht, wobei das Salz bevorzugt Natriumcromoglicat (DSCG) ist, wobei das Natriumcromoglicat bevorzugter in einer Menge von 0,01-0,1 Gewichts-%, bezogen auf das Gesamtgewicht der Formulierung, eingesetzt wird.

16. Suspensions-Aerosolformulierung nach Anspruch 10, die in einem Dosierinhalator enthalten ist, beinhaltend:
(i) 0,035% Fluticasonpropionat, 0,007% Formoterolfumarat, 0,034% Natriumcromoglicat, 1,4% Ethanol und HFA 227 q.s. ad 100, wobei alle Prozentangaben auf das Gesamtgewicht der Zusammensetzung bezogen sind und wobei die Nominaldosis von Fluticasonpropionat 50 Mikrogramm und für Formoterolfumarat 10 Mikrogramm zweimal täglich (BID) beträgt;
(ii) 0,071 % Fluticasonpropionat, 0,007% Formoterolfumarat, 0,034% Natriumcromoglicat, 1,4% Ethanol und HFA 227 q.s. ad 100, wobei alle Prozentangaben auf das Gesamtgewicht der Zusammensetzung bezogen sind und wobei die Nominaldosis von Fluticasonpropionat 100 Mikrogramm und für Formoterolfumarat 10 Mikrogramm zweimal täglich (BID) beträgt;
(iii) 0,178% Fluticasonpropionat, 0,007% Formoterolfumarat, 0,034% Natriumcromoglicat, 1,4% Ethanol und HFA 227 q.s. ad 100, wobei alle Prozentangaben auf das Gesamtgewicht der Zusammensetzung bezogen sind und wobei die Nominaldosis von Fluticasonpropionat 250 Mikrogramm und für Formoterolfumarat 10 Mikrogramm zweimal täglich (BID) beträgt;
(iv) 0,357% Fluticasonpropionat, 0,007% Formoterolfumarat, 0,034% Natriumcromoglicat, 1,4% Ethanol und HFA 227 q.s. ad 100, wobei alle Prozentangaben auf das Gesamtgewicht der Zusammensetzung bezogen sind und wobei die Nominaldosis von Fluticasonpropionat 500 Mikrogramm und für Formoterolfumarat 10 Mikrogramm zweimal täglich (BID) beträgt;
(v) 0,357% Fluticasonpropionat, 0,014% Formoterolfumarat, 0,068% Natriumcromoglicat, 1,4% Ethanol und HFA 227 q.s. ad 100, wobei alle Prozentangaben auf das Gesamtgewicht der Zusammensetzung bezogen sind und wobei die Nominaldosis von Fluticasonpropionat 500 Mikrogramm und für Formoterolfumarat 20 Mikrogramm zweimal täglich beträgt; oder
(vi) 0,357% Fluticasonpropionat, 0,014% Formoterolfumarat, 0,034% Natriumcromoglicat, 1,4% Ethanol und HFA 227 q.s. ad 100, wobei alle Prozentangaben auf das Gesamtgewicht der Zusammensetzung bezogen sind und wobei die Nominaldosis von Fluticasonpropionat 500 Mikrogramm und für Formoterolfumarat 20 Mikrogramm zweimal täglich beträgt.

17. Suspensions-Aerosolformulierung nach Anspruch 10 zur Verwendung bei der Behandlung von Asthma, allergischer Rhinitis oder chronisch-obstruktiver Lungenerkrankung (COPD) .

18. Suspensions-Aerosolformulierung nach Anspruch 17 zur gleichzeitigen, aufeinanderfolgenden oder separaten Verwendung mit einem kurzwirksamen Beta-2-Agonisten, wobei bevorzugt der kurzwirksame Beta-2-Agonist aus der Gruppe ausgewählt ist, die aus Albuterol, Salbutamol, Terbutalin, Fenoterol, Levalbuterol, Reproterol und Pirbuterol besteht.

## Revendications

1. Utilisation d'un agent mouillant dans une formulation d'aérosol en suspension comprenant des particules de fumarate de formotérol dihydraté et de propionate de fluticasone en suspension dans un propulseur HFA contenu dans un inhalateur-doseur comprenant un réservoir et une valve doseuse, pour réduire le dépôt de particules sur les surfaces du réservoir et de la valve doseuse, dans laquelle l'agent mouillant est sélectionné dans le groupe constitué de l'éthanol, du propylène glycol, du glycérol, du butanediol et de mélanges de ceux-ci, préférablement dans laquelle l'agent mouillant est l'éthanol.

2. Utilisation selon la revendication 1, dans laquelle l'agent mouillant est utilisé dans une quantité inférieure à 2 % en poids relativement au poids total de la formulation d'aérosol en suspension, préférablement de 1,99 à 0,01 % en poids relativement au poids total de la formulation d'aérosol en suspension, plus préférablement de 1,5 % à 1,0 % en poids relativement au poids total de la formulation d'aérosol en suspension.

3. Utilisation selon la revendication 1, dans laquelle le propulseur HFA est le HFA 227.

4. Utilisation selon la revendication 1, dans laquelle le fumarate de formotérol dihydraté est utilisé dans une quantité de 0,003 à 0,04 % en poids.

5. Utilisation selon la revendication 1, dans laquelle le propionate de fluticasone est utilisé dans une quantité de 0,01 à 0,6 % en poids.

6. Utilisation selon la revendication 1, dans laquelle la formulation d'aérosol en suspension comprend un sel sélectionné dans le groupe constitué du nédocromil et de l'acide cromoglycique, préférablement dans laquelle le sel est le cromoglycate de sodium (DSCG), préférablement dans laquelle le cromoglycate de sodium est utilisé dans une quantité de 0,01 à 0,1 % en poids relativement au poids total de la formulation.

7. Utilisation selon la revendication 1, dans laquelle la formulation d'aérosol en suspension, lorsqu'elle est distribuée à partir de l'inhalateur-doseur, apporte une dose du fumarate de formotérol dihydraté ainsi que du propionate de fluticasone qui a une variance ne dépassant pas +/- 15 % de la dose administrée moyenne cible (et pas plus de 1 valeur en dehors de +/- 25 % de la cible et aucune valeur en dehors de +/- 30 % de la cible) lorsque la formulation est conservée à 25 degrés centigrade et à 60 % d'humidité relative, plus particulièrement à 40 degrés centigrade et à 75 % d'humidité relative sur une période de 1 mois au maximum, plus particulièrement de 3 mois au maximum, encore plus particulièrement de 6 mois au maximum.

8. Utilisation selon la revendication 1, dans laquelle la formulation d'aérosol en suspension, lorsqu'elle est conservée dans l'inhalateur-doseur pendant au moins 2 semaines et à une température de 17 à 25 degrés centigrade et une humidité relative de 29 à 63 %, a une fraction granulométrique fine moyenne de 30 à 60 % de la dose nominale comme représentation *in vitro* de la distribution granulométrique aérodynamique.

9. Utilisation selon la revendication 1, dans laquelle la formulation d'aérosol en suspension contenue dans l'inhalateur-doseur comprend :
(i) 0,035 % de propionate de fluticasone, 0,007 % de fumarate de formotérol, 0,034 % de cromoglycate de sodium, 1,4 % d'éthanol et du HFA 227 en quantité suffisante pour obtenir 100 %, tous les pourcentages étant exprimés relativement au poids total de la composition, la dose nominale de propionate de fluticasone étant de 50 microgrammes, et la dose nominale de fumarate de formotérol étant de 10 microgrammes, deux fois par jour (bid) ;
(ii) 0,071 % de propionate de fluticasone, 0,007 % de fumarate de formotérol, 0,034 % de cromoglycate de sodium, 1,4 % d'éthanol et du HFA 227 en quantité suffisante pour obtenir 100 %, tous les pourcentages étant exprimés relativement au poids total de la composition, la dose nominale de propionate de fluticasone étant de 100 microgrammes, et la dose nominale de fumarate de formotérol étant de 10 microgrammes, deux fois par jour (bid) ;
(iii) 0,178 % de propionate de fluticasone, 0,007 % de fumarate de formotérol, 0,034 % de cromoglycate de sodium, 1,4 % d'éthanol et du HFA 227 en quantité suffisante pour obtenir 100 %, tous les pourcentages étant exprimés relativement au poids total de la composition, la dose nominale de propionate de fluticasone étant de 250 microgrammes, et la dose nominale de fumarate de formotérol étant de 10 microgrammes, deux fois par jour (bid) ;
(iv) 0,357 % de propionate de fluticasone, 0,007 % de fumarate de formotérol, 0,034 % de cromoglycate de sodium, 1,4 % d'éthanol et du HFA 227 en quantité suffisante pour obtenir 100 %, tous les pourcentages étant exprimés relativement au poids total de la composition, la dose nominale de propionate de fluticasone étant de 500 microgrammes, et la dose nominale de fumarate de formotérol étant de 10 microgrammes, deux fois par jour (bid) ;
(v) 0,357 % de propionate de fluticasone, 0,014 % de fumarate de formotérol, 0,068 % de cromoglycate de sodium, 1,4 % d'éthanol et du HFA 227 en quantité suffisante pour obtenir 100 %, tous les pourcentages étant exprimés relativement au poids total de la composition, la dose nominale de propionate de fluticasone étant de 500 microgrammes, et la dose nominale de fumarate de formotérol étant de 20 microgrammes, deux fois par jour ; ou
(vi) 0,357 % de propionate de fluticasone, 0,014 % de fumarate de formotérol, 0,034 % de cromoglycate de sodium, 1,4 % d'éthanol et du HFA 227 en quantité suffisante pour obtenir 100 %, tous les pourcentages étant exprimés relativement au poids total de la composition, la dose nominale de propionate de fluticasone étant de 500 microgrammes, et la dose nominale de fumarate de formotérol étant de 20 microgrammes, deux fois par jour.

10. Formulation d'aérosol en suspension comprenant des particules de fumarate de formotérol dihydraté et de propionate de fluticasone en suspension dans un propulseur HFA et un agent mouillant, dans laquelle l'agent mouillant est sélectionné dans le groupe constitué de l'éthanol, du propylène glycol, du glycérol, du butanediol et de mélanges de ceux-ci, et est utilisé dans une quantité inférieure à 2 % en poids relativement au poids total de la formulation, et dans laquelle les particules ont une distribution granulométrique dans laquelle D10 (10 % de la distribution volumique) est de 0,2 à 2 micromètres, D50 est de 1 à 4 micromètres et D90 est de 2 à 6 micromètres lorsque ces valeurs sont mesurées par diffraction laser, soit en suspension, soit sous la forme d'une poudre sèche dispersée.

11. Formulation d'aérosol en suspension selon la revendication 10, dans laquelle l'agent mouillant est utilisé dans une quantité de 1,99 à 0,01 % en poids relativement au poids total de la formulation d'aérosol en suspension, préférablement dans une quantité de 1,5 % à 1 % en poids relativement au poids total de la formulation d'aérosol en suspension.

12. Formulation d'aérosol en suspension selon la revendication 10, dans laquelle le propulseur HFA est le HFA 227.

13. Formulation d'aérosol en suspension selon la revendication 10, dans laquelle le fumarate de formotérol dihydraté est utilisé dans une quantité de 0,003 à 0,04 % en poids.

14. Formulation d'aérosol en suspension selon la revendication 10, dans laquelle le propionate de fluticasone est utilisé dans une quantité de 0,01 à 0,6 % en poids.

15. Formulation d'aérosol en suspension selon la revendication 10, la formulation d'aérosol en suspension comprenant en outre un sel sélectionné dans le groupe constitué du nédocromil et de l'acide cromoglycique, préférablement dans laquelle le sel est le cromoglycate de sodium (DSCG), plus préférablement dans laquelle le cromoglycate de sodium est utilisé dans une quantité de 0,01 à 0,1 % en poids relativement au poids total de la formulation.

16. Formulation d'aérosol en suspension selon la revendication 10, contenue dans un inhalateur-doseur comprenant :
(i) 0,035 % de propionate de fluticasone, 0,007 % de fumarate de formotérol, 0,034 % de cromoglycate de sodium, 1,4 % d'éthanol et du HFA 227 en quantité suffisante pour obtenir 100 %, tous les pourcentages étant exprimés relativement au poids total de la composition, la dose nominale de propionate de fluticasone étant de 50 microgrammes, et la dose nominale de fumarate de formotérol étant de 10 microgrammes, deux fois par jour (bid) ;
(ii) 0,071 % de propionate de fluticasone, 0,007 % de fumarate de formotérol, 0,034 % de cromoglycate de sodium, 1,4 % d'éthanol et du HFA 227 en quantité suffisante pour obtenir 100 %, tous les pourcentages étant exprimés relativement au poids total de la composition, la dose nominale de propionate de fluticasone étant de 100 microgrammes, et la dose nominale de fumarate de formotérol étant de 10 microgrammes, deux fois par jour (bid) ;
(iii) 0,178 % de propionate de fluticasone, 0,007 % de fumarate de formotérol, 0,034 % de cromoglycate de sodium, 1,4 % d'éthanol et du HFA 227 en quantité suffisante pour obtenir 100 %, tous les pourcentages étant exprimés relativement au poids total de la composition, la dose nominale de propionate de fluticasone étant de 250 microgrammes, et la dose nominale de fumarate de formotérol étant de 10 microgrammes, deux fois par jour (bid) ;
(iv) 0,357 % de propionate de fluticasone, 0,007 % de fumarate de formotérol, 0,034 % de cromoglycate de sodium, 1,4 % d'éthanol et du HFA 227 en quantité suffisante pour obtenir 100 %, tous les pourcentages étant exprimés relativement au poids total de la composition, la dose nominale de propionate de fluticasone étant de 500 microgrammes, et la dose nominale de fumarate de formotérol étant de 10 microgrammes, deux fois par jour (bid) ;
(v) 0,357 % de propionate de fluticasone, 0,014 % de fumarate de formotérol, 0,068 % de cromoglycate de sodium, 1,4 % d'éthanol et du HFA 227 en quantité suffisante pour obtenir 100 %, tous les pourcentages étant exprimés relativement au poids total de la composition, la dose nominale de propionate de fluticasone étant de 500 microgrammes, et la dose nominale de fumarate de formotérol étant de 20 microgrammes, deux fois par jour ; ou
(vi) 0,357 % de propionate de fluticasone, 0,014 % de fumarate de formotérol, 0,034 % de cromoglycate de sodium, 1,4 % d'éthanol et du HFA 227 en quantité suffisante pour obtenir 100 %, tous les pourcentages étant exprimés relativement au poids total de la composition, la dose nominale de propionate de fluticasone étant de 500 microgrammes, et la dose nominale de fumarate de formotérol étant de 20 microgrammes, deux fois par jour.

17. Formulation d'aérosol en suspension selon la revendication 10, destinée à une utilisation dans le traitement d'un asthme, d'une rhinite allergique ou d'une bronchopneumopathie chronique obstructive (BPCO).

18. Formulation d'aérosol en suspension selon la revendication 17, destinée à une utilisation simultanément, séquentiellement ou séparément avec un bêta-2-agoniste à courte durée d'action, le bêta-2-agoniste à courte durée d'action étant préférablement sélectionné dans le groupe constitué de l'albutérol, du salbutamol, de la terbutaline, du fénotérol, du levalbutérol, du reprotérol et du pirbutérol.
